# EUROPEAN PATENT SPECIFICATION

(11) **EP 1 394 547 B1**
(45) Date of publication and mention of the grant of the patent: **12.04.2006**
(21) Application number: 03019459.1
(22) Date of filing: 28.08.2003
(51) Int. Cl.: G01N 33/543

(54) **Chemical luminescence method using biochemical analysis units**
Chemische Lumineszenzverfahren mit Biochemischeanalyse-Einheiten
Procédé de luminescence chimique en employant des unités d'analyse biochimique

(30) Priority: 29.08.2002 JP 2002250760
(43) Date of publication of application: 03.03.2004
(73) Proprietor: FUJI PHOTO FILM CO., LTD., Minami-Ashigara-shi, Kanagawa-ken (JP)
(72) Inventor: Nakajima, Kenji, c/o Fuji Photo Film Co., Ltd., Minamiashigara-shi, Kanagawa-ken (JP)
(74) Representative: Klunker . Schmitt-Nilson . Hirsch

(56) References cited:
- EP-A- 1 333 283
- EP-A- 1 333 284
- WO-A-00/45950
- WO-A-98/29736
- WO-A-02/094846
- WO-A-03/005013

## Description

### BACKGROUND OF THE INVENTION

### Field of the Invention

This invention relates to a chemical luminescence method for detecting a labeled receptor or a labeled ligand with a chemical luminescence technique. This invention particularly relates to a chemical luminescence method, wherein a labeled receptor or a labeled ligand is detected with a chemical luminescence technique by the utilization of a biochemical analysis unit provided with porous adsorptive regions.

### Description of the Related Art

With micro array analysis systems and macro array analysis systems, liquids containing specific binding substances (i.e., the substances, which are capable of specifically binding to organism-originating substances and whose base sequences, base lengths, compositions, characteristics, and the like, are known) are spotted onto different positions on a surface of a biochemical analysis unit, such as a membrane filter, a plurality of spot-shaped regions are thereby formed on the surface of the biochemical analysis unit. Examples of the specific binding substances include hormones, tumormarkers, enzymes, antibodies, antigens, abzymes, other proteins, nucleic acids, cDNA's, DNA's, and RNA's. Thereafter, an organism-originating substance, which has been labeled with a radioactive labeling substance, a fluorescent labeling substance, a labeling substance capable of producing chemical luminescence when being brought into contact with a chemical luminescence substrate, or the like, is subjected to hybridization, or the like, with the specific binding substances, which are contained in the spot-shaped regions of the biochemical analysis unit. The organism-originating substance is thus specifically bound to one of the specific binding substances, which are contained in the spot-shaped regions of the biochemical analysis unit. The organism-originating substance is the substance, which has been sampled from an organism through extraction, isolation, or the like, or has been subjected to chemical treatment after being sampled, and which has been labeled with the radioactive labeling substance, the fluorescent labeling substance, the labeling substance capable of producing the chemical luminescence when being brought into contact with a chemical luminescence substrate, or the like. Examples of the organism-originating substances include hormones, tumor markers, enzymes, antibodies, antigens, abzymes, other proteins, nucleic acids, DNA's, and mRNA's.

In cases where the organism-originating substance has been labeled with the radioactive labeling substance, a stimulable phosphor layer of a stimulable phosphor sheet is then exposed to radiation radiated out from the radioactive labeling substance, which is contained selectively in the spot-shaped region of the biochemical analysis unit. Thereafter, the stimulable phosphor layer is exposed to stimulating rays, which cause the stimulable phosphor layer to emit light in proportion to the amount of energy stored on the stimulable phosphor layer during the exposure of the stimulable phosphor layer to the radiation. The light emitted by the stimulable phosphor layer is photoelectrically detected, and data for a biochemical analysis is thereby obtained.

In cases where the organism-originating substance has been labeled with the fluorescent labeling substance, excitation light is irradiated to the spot-shaped regions of the biochemical analysis unit, and the fluorescent labeling substance, which is contained selectively in the spot-shaped region of the biochemical analysis unit, is excited by the excitation light to produce fluorescence. The thus produced fluorescence is photoelectrically detected, and data for a biochemical analysis is thereby obtained.

In cases where the organism-originating substance has been labeled with the labeling substance capable of producing the chemical luminescence when being brought into contact with a chemical luminescence substrate, the labeling substance, which is contained selectively in the spot-shaped region of the biochemical analysis unit, is brought into contact with the chemical luminescence substrate. Also, the chemical luminescence produced by the labeling substance is photoelectrically detected, and data for a biochemical analysis is thereby obtained.

Heretofore, with the analysis systems utilizing the biochemical analysis units, the hybridization, or the like, has ordinarily been performed with a shaking technique. With the shaking technique, the experimenter manually puts a biochemical analysis unit, on which specific binding substances have been fixed, into a hybridization bag and adds a reaction liquid, which contains an organism-originating substance having been labeled, into the hybridization bag. Also, the experimenter manually gives vibrations to the hybridization bag, and the labeled organism-originating substance is thus moved through convection or diffusion. In this manner, the labeled organism-originating substance is specifically bound to one of the specific binding substances having been fixed on the biochemical analysis unit. Thereafter, the biochemical analysis unit is taken out from the hybridization bag and washed in a vessel filled with a washing liquid.

However, with the shaking technique described above, it is not always possible to bring the hybridization reaction liquid into uniform contact with the plurality of spot-shaped regions, which contain the specific binding substances. Therefore, the problems occur in that the specific binding substances and the labeled organism-originating substance cannot efficiently be subjected to the hybridization.

Also, with the shaking technique described above, the problems occur in that the results of the hybridization vary for different experimenters, and reproducibility becomes low. Further, there is the risk that the reproducibility becomes low even when the hybridization is performed by an identical experimenter.

In order to solve the problems described above, the applicant proposed a method defined by claim 1, wherein a reaction liquid containing a labeled organism-originating substance is forcibly caused to flow through adsorptive regions of a biochemical analysis unit, and the labeled organism-originating substance is thus caused to penetrate sufficiently into the interiors of the adsorptive regions of the biochemical analysis unit, wherein the forced flowing is ceased during a period of time longer than the period of time during which the reaction liquid containing said substance has been forced to flow. The proposed method is described in Japanese Patent Application No. 2002-26816.

With the detection method utilizing the chemical luminescence technique, in which the organism-originating substance is detected by use of the labeling substance capable of producing the chemical luminescence when being brought into contact with a chemical luminescence substrate, it is necessary to perform the procedures described below. Specifically, the labeled organism-originating substance (hereinbelow referred to as the labeled receptor or the labeled ligand in the field of the chemical luminescence technique) is subjected to specific binding with the specific binding substance (hereinbelow referred to as the ligand or the receptor in the field of the chemical luminescence technique). Also, an antibody with respect to the substance, such as an antigen, with which the labeled receptor or the labeled ligand has been labeled, is labeled with the labeling substance, such as an enzyme, which is capable of producing the chemical luminescence. (The thus labeled antibody will hereinbelow be referred to as the enzyme-labeled antibody.; After the labeled receptor or the labeled ligand has been specifically bound to the ligand or the receptor in the manner described above, the enzyme-labeled antibody is subjected to specific binding with the antigen of the labeled receptor or the labeled ligand. Thereafter, the enzyme-labeled antibody, which has been specifically bound to the antigen of the labeled receptor or the labeled ligand, and the chemical luminescence substrate, which is capable of undergoing specific binding with the enzyme of the enzyme-labeled antibody, are brought into contact with each other.

With the conventional chemical luminescence technique, besides the procedures for the biochemical analysis unit utilizing the fluorescent labeling substance or the radioactive labeling substance, it is necessary that the procedures described below be performed. Specifically, the experimenter manually puts the biochemical analysis unit, in which the labeled receptor or the labeled ligand has been bound to the adsorptive region, again into a hybridization bag. Also, the experimenter manually adds the reaction liquid, which contains the enzyme-labeled antibody capable of undergoing specific reaction with the antigen of the labeled receptor or the labeled ligand, into the hybridization bag. Further, the experimenter manually gives vibrations to the hybridization bag, and the enzyme-labeled antibody is thus moved through convection or diffusion. In this manner, the enzyme-labeled antibody is specifically bound to the labeled receptor or the labeled ligand. Thereafter, the chemical luminescence substrate is brought into contact with the enzyme-labeled antibody having been specifically bound to the labeled receptor or the labeled ligand and is caused to react with the enzyme-labeled antibody.

However, the conventional chemical luminescence technique utilizing the shaking technique has the problems described below. Specifically, the enzyme-labeled antibody cannot suff icientlypenetrate into the interior of the adsorptive region of the biochemical analysis unit. Therefore, a ratio of the intensity of the chemical luminescence, which intensity corresponds to the amount of the labeled receptor or the labeled ligand having been bound to the adsorptive region, to the intensity of the luminescence (noise or background) of an adsorptive region, to which the labeled receptor or the labeled ligand has not been bound, cannot be kept high. (The signal-to-noise ratio of a signal representing the intensity of the chemical luminescence, which intensity corresponds to the amount of the labeled receptor or the labeled ligand having been bound to the adsorptive region, to the noise or the background cannot be kept high.) Accordingly, in cases where the amount of the labeled receptor or the labeled ligand, which is bound to the adsorptive region, is small (1picogramor less), it becomes difficult for the labeled receptor or the labeled ligand to be detected.

There exists prior art which has been published after the priority date of the present application (EP-A-1 333 284 and EP-A-1 333 283). This prior art is related to a chemical luminescence method having the features i)-iv) of each of claims 1 and 2. These prior art references, however, do not disclose specific features regarding the time period in which the force flowing is going on.

### SUMMARY OF THE INVENTION

The primary object of the present invention is to provide a chemical luminescence method using a biochemical analysis unit, wherein an enzyme-labeled antibody is capable of being efficiently bound to a labeled receptor or a labeled ligand, which has been bound to a ligand or a receptor having been fixed to a biochemical analysis unit, and wherein data is capable of being obtained with good reproducibility and good quantitative characteristics.

The present invention provides a first chemical luminescence method using a biochemical analysis unit, comprising the steps defined by claim 1.

The first chemical luminescence method using a biochemical analysis unit in accordance with the present invention comprises the feature that, after the reaction liquid containing the enzyme-labeled antibody has been forcibly caused to flow such that the reaction liquid flows across each of the porous adsorptive regions of the biochemical analysis unit, the forced flowing is ceased during a period of time longer than the period of time during which the reaction liquid containing the enzyme-labeled antibody has been forced to flow. In such cases, after the reaction liquid containing the enzyme-labeled antibody has been forced to flow, the forced flowing may be ceased consecutively during the period of time longer than the period of time during which the reaction liquid containing the enzyme-labeled antibody has been forced to flow. Alternatively, a cycle comprising the forced flowing of the reaction liquid, which contains the enzyme-labeled antibody, and the ceasing of the flowing of the reaction liquid may be iterated, such that, as a whole, the forced flowing is ceased during the period of time longer than the period of time during which the reaction liquid containing the enzyme-labeled antibody has been forced to flow.

The present invention also provides a second chemical luminescence method using a biochemical analysis unit, comprising the steps of claim 2.

The second chemical luminescence method using a biochemical analysis unit in accordance with the present invention comprises the feature that, after the reaction liquid containing the enzyme-labeled antibody has been forced to flow such that the reaction liquid flows across each of the porous adsorptive regions of the biochemical analysis unit, the forced flowing is ceased during a period of time longer than the period of time during which the reaction liquid containing the enzyme-labeled antibody has been forced to flow.

With the first chemical luminescence method using a biochemical analysis unit in accordance with the present invention, at the time at which the enzyme-labeled antibody is subjected to the specific binding with the labeled receptor or the labeled ligand, which has been specifically bound to at least one of the ligands or at least one of the receptors, the reaction liquid containing the enzyme-labeled antibody is forced to flow such that the reaction liquid containing the enzyme-labeled antibody flows across each of the porous adsorptive regions of the biochemical analysis unit. Therefore, the enzyme-labeled antibody is capable of being caused to penetrate sufficiently into the interior of each of the adsorptive regions. Accordingly, the limit of detection of the labeled receptor or the labeled ligand is capable of being enhanced. Also, in cases where the amount of the labeled receptor or the labeled ligand is small, the labeled receptor or the labeled ligand is capable of being detected with a high signal-to-noise ratio. Therefore, the labeled receptor or the labeled ligand is capable of being detected accurately, and data is capable of being obtained with good reproducibility and good quantitative characteristics.

With the first chemical luminescence method using a biochemical analysis unit in accordance with the present invention (andwith the second chemical luminescence method using a biochemical analysis unit in accordance with the present invention), after the reaction liquid containing the enzyme-labeled antibody has been forcibly caused to flow such that the reaction liquid flows across each of the porous adsorptive regions of the biochemical analysis unit, the forcible flowing may be ceased during the period of time longer than the period of time during which the reaction liquid containing the enzyme-labeled antibody has been forcibly caused to flow. In such cases, the limit of detection of the labeled receptor or the labeled ligand is capable of being enhanced even further, and the labeled receptor or the labeled ligand is capable of being detected with a high signal-to-noise ratio.

With the second chemical luminescence method using a biochemical analysis unit in accordance with the present invention, at the time at which the labeled receptor or the labeled ligand having been labeled with the labeling substance is subjected to the specific binding with the ligands or the receptors, each of which has been bound to one of the porous adsorptive regions of the biochemical analysis unit, the reaction liquid containing the labeled receptor or the labeled ligand, which has been labeled with the labeling substance, is forcibly caused to flow such that the reaction liquid containing the labeled receptor or the labeled ligand flows across each of the porous adsorptive regions of the biochemical analysis unit. Also, at the time at which the enzyme-labeled antibody is subjected to the specific binding with the labeled receptor or the labeled ligand, which has been specifically bound to at least one of the ligands or at least one of the receptors, the reaction liquid containing the enzyme-labeled antibody is forcibly caused to flow such that the reaction liquid containing the enzyme-labeled antibody flows across each of the porous adsorptive regions of the biochemical analysis unit. Therefore, the labeled receptor or the labeled ligand and the enzyme-labeled antibody are capable of being caused to penetrate sufficiently into the interior of each of the adsorptive regions. Accordingly, in cases where the amount of the labeled receptor or the labeled ligand is small, the labeled receptor or the labeled ligand is capable of being detected with a high signal-to-noise ratio.

### BRIEF DESCRIPTION OF THE DRAWINGS

Figure 1 is a schematic perspective view showing an example of a biochemical analysis unit, which is employed in the chemical luminescence method using a biochemical analysis unit in accordance with the present invention,
Figure 2 is a schematic sectional view showing an example of a reactor, which is employed for the chemical luminescence method using a biochemical analysis unit in accordance with the present invention, and
Figure 3 is a schematic sectional view showing a different example of a reactor, which is employed for the chemical luminescence method using a biochemical analysis unit in accordance with the present invention.

### DESCRIPTION OF THE PREFERRED EMBODIMENTS

The present invention will hereinbelow be described in further detail with reference to the accompanying drawings.

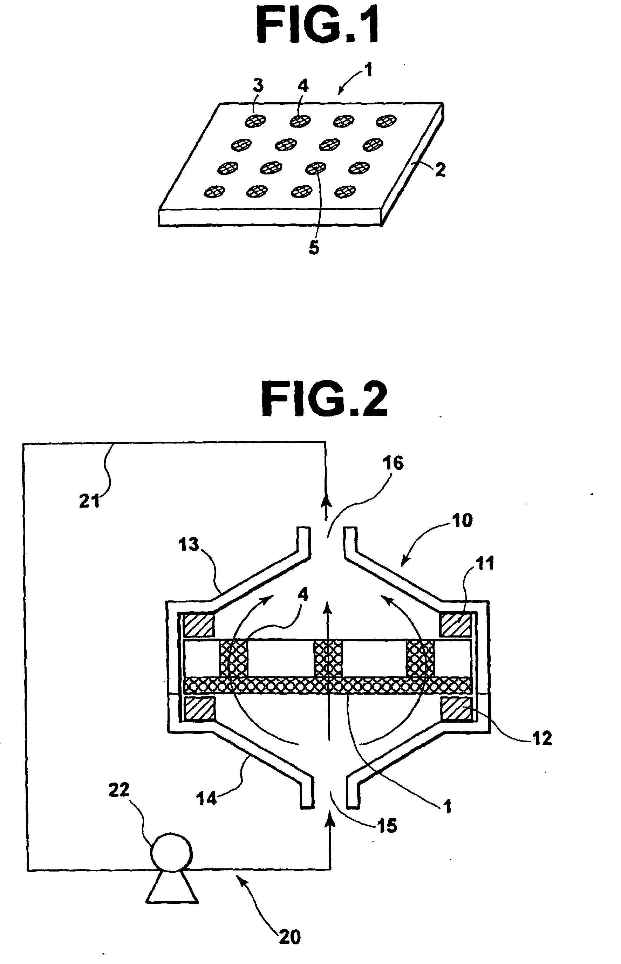

Figure 1 is a schematic perspective view showing an example of a biochemical analysis unit, which is employed in the chemical luminescence method using a biochemical analysis unit in accordance with the present invention. With reference to Figure 1, a biochemical analysis unit 1 comprises a base plate 2, which is provided with a plurality of holes 3, 3, ..., and a plurality of adsorptive regions 4, 4, ..., each of which is filled in one of the holes 3, 3, ... and comprises a porous material adhered to the base plate 2. Each of ligands or receptors, whose structures or characteristics are known, has been spotted onto one of the adsorptive regions 4, 4, ... and has then been immobilized with treatment.

Such that light scattering may be prevented from occurring within the biochemical analysis unit 1, the base plate 2 should preferably be made from a material, which does not transmit light or which attenuates light. The material for the formation of the base plate 2 should preferably be a metal or a ceramic material. Also, in cases where a plastic material, for which the hole making processing is capable of being performed easily, is employed as the material for the formation of the base plate 2, particles should preferably be dispersed within the plastic material, such that light is capable of being attenuated even further.

Examples of the metals, which may be utilized preferably for the formation of the base plate 2, include copper, silver, gold, zinc, lead, aluminum, titanium, tin, chromium, iron, nickel, cobalt, tantalum, and alloys, such as stainless steel and bronze. Examples of the ceramic materials, which may be utilized preferably for the formation of the base plate 2, include alumina, zirconia, magnesia, and quartz. Examples of the plastic materials, which may be utilized preferably for the formation of the base plate 2, include polyolefins, such as a polyethylene and a polypropylene; polystyrenes; acrylic resins, such as a polymethyl methacrylate; polyvinyl chlorides; polyvinylidene chlorides; polyvinylidene fluorides; polytetrafluoroethylenes; polychlorotrifluoroethylenes; polycarbonates; polyesters, such as a polyethylene naphthalate and a polyethylene terephthalate; aliphatic polyamides, such as a 6-nylon and a 6,6-nylon; polyimides; polysulfones; polyphenylene sulfides; silicon resins, such as a polydiphenyl siloxane; phenolic resins, such as novolak; epoxy resins; polyurethanes; celluloses, such as cellulose acetate and nitrocellulose; copolymers, such as a butadiene-styrene copolymer; and blends of plastic materials.

Such that the density of the holes 3, 3, ... made through the base plate 2 may be enhanced, the area (size) of the opening of each of the holes 3, 3, ... may ordinarily be smaller than 5mm². The area of the opening of each of the holes 3, 3, ... should preferably be smaller than 1mm², should more preferably be smaller than 0.3mm², and should most preferably be smaller than 0.01mm². Also, the area of the opening of each of the holes 3, 3, ... should preferably be at least 0.001mm².

The pitch of the holes 3, 3, ... (i.e., the distance between the center points of two holes which are adjacent to each other) should preferably fall within the range of 0.05mm to 3mm. Also, the spacing between two adjacent holes 3, 3 (i.e., the shortest distance between edges of two adjacent holes 3, 3) should preferably fall within the range of 0.01mm to 1.5mm. Thenumber (thearraydensity) oftheholes3, 3, ... mayordinarily be at least 10 holes/cm². The number (the array density) of the holes 3, 3, ... should preferably be at least 100 holes/cm², should more preferably be at least 500 holes/cm², and should most preferably be at least 1,000 holes/cm². Also, the number (the array density) of the holes 3, 3, ... should preferably be at most 100, 000 holes/cm², and should more preferably be at most 10, 000 holes/cm². The holes 3, 3, ... need not necessarily be arrayed at equal spacing as illustrated in Figure 1. For example, the holes 3, 3, ... may be grouped into several number of blocks (units) comprising a plurality of holes and may be formed in units of the blocks.

In the chemical luminescence method using a biochemical analysis unit in accordance with the present invention, as the porous material for the formation of the adsorptive regions of the biochemical analysis unit, a porous quality material or a fiber material may be utilized preferably. The porous quality material and the fiber material may be utili zed in combination in order to form the adsorptive regions of the biochemical analysis unit. In the chemical luminescence method using a biochemical analysis unit in accordance with the present invention, the porous material, which may be utilized for the formation of the adsorptive regions of the biochemical analysis unit, may be an organic material, an inorganic material, or an organic-inorganic composite material.

The organic porous quality material, which may be utilized for the formation of the adsorptive regions of the biochemical analysis unit, may be selected from a wide variety of materials. However, the organic porous quality material should preferably be a carbon porous quality material, such as active carbon, or a porous quality material capable of forming a membrane filter. As the porous quality material capable of forming a membrane filter, a polymer soluble in a solvent should preferably be utilized. Examples of the polymers soluble in a solvent include cellulose derivatives, such as nitrocellulose, regenerated cellulose, cellulose acetate, and cellulose acetate butyrate; aliphatic polyamides, such as a 6-nylon, a 6, 6-nylon, and a 4,10-nylon; polyolefins, such as a polyethylene and a polypropylene; chlorine-containing polymers, such as a polyvinyl chloride and a polyvinylidene chloride; fluorine resins, such as a polyvinylidene fluoride and a polytetrafluoride; polycarbonates; polysulfones; alginic acids and alginic acid derivatives, such as alginic acid, calcium alginate, and an alginic acid-polylysine polyion complex; and collagen. Copolymers or composite materials (mixture materials) of the above-enumerated polymers may also be utilized.

The fiber material, which may be utilized for the formation of the adsorptive regions of the biochemical analysis unit, may be selected f rom a wide variety of materials. Examples of the fiber materials, whichmay be utilized preferably, include the cellulose derivatives and the aliphatic polyamides enumerated above.

The inorganic porous quality material, which may be utilized for the formation of the adsorptive regions of the biochemical analysis unit, may be selected from a wide variety of materials. Examples of the inorganic porous quality materials, which may be utilized preferably, include metals, such as platinum, gold, iron, silver, nickel, and aluminum; oxides of metals, and the like, such as alumina, silica, titania, and zeolite; metal salts, such as hydroxyapatite and calcium sulfate; and composite materials of the above-enumerated materials.

Perforation of the plurality of the holes 3, 3, ... through the base plate 2 may be performed with, for example, a punching technique for punching with a pin, a technique for electrical discharge machining, in which a pulsed high voltage is applied across electrodes in order to volatilize the base plate material, an etching technique, or a laser beam irradiation technique. In cases where the material of the base plate is a metal material or a plastic material, the biochemical analysis unit may be prepared with an operation for performing corona discharge or plasma discharge on the surface of the base plate, applying an adhesive agent to the surface of the base plate, and laminating the porous material for the formation of the adsorptive regions by use of means, such as a press. At the time of the lamination, the porous material for the formation of the adsorptive regions may be heated and softened, such that the adsorptive regions may be forced easily within the holes. Also, in cases where the porous material for the formation of the adsorptive regions is pressed against the base plate, the base plate and the porous material for the formation of the adsorptive regions may be divided previously into a plurality of sheets, and the plurality of the sheets may be pressed intermittently. Alternatively, a long web of the base plate and a long web of the porous material for the formation of the adsorptive regions may be conveyed continuously between two rolls.

In the chemical luminescence method using a biochemical analysis unit in accordance with the present invention, the biochemical analysis unit having been prepared by use of the material and the technique described above may be utilized. Alternatively, a commercially available biochemical analysis unit may be utilized. It is also possible to utilize a biochemical analysis unit, in which the ligands or the receptors have already been bound respectively to the porous adsorptive regions.

Figure 2 is a schematic sectional view showing an example of a reactor (a reaction apparatus), which is employed for the chemical luminescence method using a biochemical analysis unit in accordance with the present invention. With reference to Figure 2, the reactor comprises a reaction vessel 10 and flowing means 20. The reaction vessel 10 comprises a reaction vessel upper half 13 and a reaction vessel lower half 14. The reaction vessel upper half 13 is releasably secured to the reaction vessel lower half 14. Also, the reaction vessel 10 is provided with a support section for releasably supporting the biochemical analysis unit 1 within the reaction vessel 10, the biochemical analysis unit 1 being provided with the plurality of the porous adsorptive regions, to which the ligands or the receptors have been bound respectively. The support section comprises an upper support piece 11 and a lower support piece 12. When the biochemical analysis unit 1 is to be set within the reaction vessel 10, the reaction vessel upper half 13 is dismounted from the reaction vessel lower half 14, and the biochemical analysis unit 1 is set on the lower support piece 12. A bottom wall of the reaction vessel lower half 14 is provided with a reaction liquid inlet 15, through which a reaction liquid is capable of flowing. Also, a top wall of the reaction vessel upper half 13 is provided with a reaction liquid outlet 16, through which the reaction liquid is capable of flowing.

The flowing means 20 comprises a reaction liquid circulating pipe 21 and a pump 22. One end of the reaction liquid circulating pipe 21 is releasably fitted to the reaction liquid inlet 15 of the reaction vessel 10. The other end of the reaction liquid circulating pipe 21 is releasably fitted to the reaction liquid outlet 16 of the reaction vessel 10. The reaction liquid is introduced by the pump 22 into the reaction vessel 10 through the reaction liquid inlet 15. Within the reaction vessel 10, the reaction liquid is forcibly caused to flow such that the reaction liquid flows across each of the adsorptive regions 4, 4, ... of the biochemical analysis unit 1. Thereafter, the reaction liquid is discharged through the reaction liquid outlet 16, passes through the reaction liquid circulating pipe 21, and circulates through the reaction vessel 10.

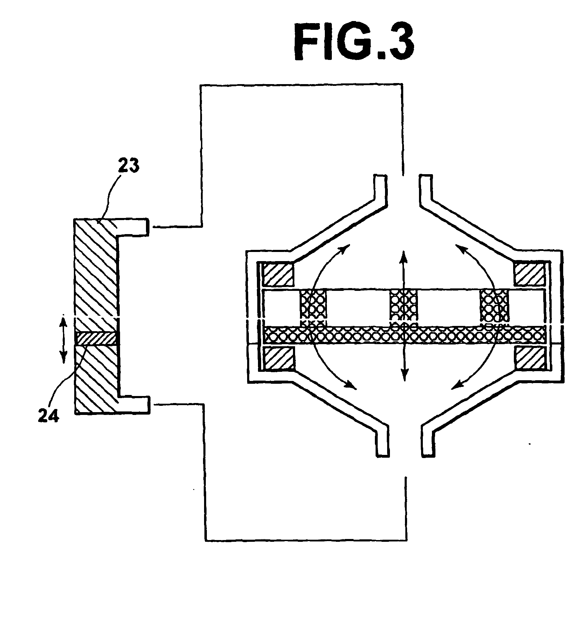

Figure 3 is a schematic sectional view showing a different example of a reactor, which is employed for the chemical luminescence method using a biochemical analysis unit in accordance with the present invention. The reactor illustrated in Figure 3 is constituted basically in the same manner as that for the reactor of Figure 2, except that a syringe 23 and a piston 24 are utilized in lieu of the pump 22, and the reaction liquid is caused to undergo reciprocal flowing within the reaction vessel. As in the cases of the reactor illustrated in Figure 3, the forcible flowing of the reaction liquid may be performed such that the reaction liquid flows reciprocally through each of the adsorptive regions 4, 4, ... of the biochemical analysis unit 1.

In the examples of the reactors illustrated in Figure 2 and Figure 3, the reaction liquid is circulated through the biochemical analysis unit 1. Alternatively, a reactor may be utilized, in which the reaction liquid merely passes through the biochemical analysis unit 1 from below (or from above) and is not circulated.

How the chemical luminescence method using a biochemical analysis unit in accordance with the present invention is performed will be described hereinbelow.

In the chemical luminescence method using a biochemical analysis unit in accordance with the present invention, firstly, the ligands or the receptors are bound respectively to the adsorptive regions of the biochemical analysis unit, which is provided with the plurality of the porous adsorptive regions.

Examples of the ligands or the receptors, which are bound respectively to the porous adsorptive regions of the biochemical analysis unit, include hormones, tumor markers, enzymes,antibodies,antigens, abzymes, other proteins,nucleic acids, cDNA's, DNA's, and RNA's, whose characteristics, compositions, structures, base sequences, base lengths, and the like, are known. After the ligands or the receptors have been spotted respectively onto the adsorptive regions of the biochemical analysis unit, the ligands or the receptors are capable of being fixed to the adsorptive regions with ultraviolet light irradiation, or the like. In cases where the aforesaid biochemical analysis unit, in which the ligands or the receptors have already been bound respectively to the porous adsorptive regions, is utilized, the steps of spotting and fixing the ligands or the receptors are omitted.

Thereafter, a labeled receptor or a labeled ligand, which has been labeled with a labeling substance, is subjected to specific binding with the ligands or the receptors, each of which has been bound to one of the porous adsorptive regions of the biochemical analysis unit. The labeled receptor or the labeled ligand is thus specifically bound to at least one of the ligands or at least one of the receptors. The labeled receptor or the labeled ligand is the substance, which has been sampled from an organism through extraction, isolation, or the like, or has been subjected to chemical treatment after being sampled, and which has been labeled with the labeling substance. The labeled receptor or the labeled ligand is capable of undergoing the specific binding with at least one of the ligands, each of which has been bound to one of the porous adsorptive regions of the biochemical analysis unit, or at least one of the receptors, each of which has been bound to one of the porous adsorptive regions of the biochemical analysis unit. Examples of the labeled receptors or the labeled ligands include hormones, tumor markers, enzymes, antibodies, antigens, abzymes, other proteins, nucleic acids, DNA's, and mRNA's. Examples of preferable labeling substances, with which the receptors or the ligands may be labeled, include antigens, such as digoxigenin, biotin, avidin, and fluorescein, and antibodies with respect to the above-enumerated antigens.

Problems should preferably be prevented from occurring in that, instead of the labeled receptor or the labeled ligand being subjected to the specific binding with the ligands or the receptors, which have been bound respectively to the porous adsorptive regions, the labeled receptor or the labeled ligand is directly bound or adsorbed to the adsorptive regions of the biochemical analysis unit. For such purposes, before the reaction liquid containing the labeled receptor or the labeled ligand is brought into contact with the biochemical analysis unit, a blocking agent should preferably be brought into contact with the adsorptive regions of the biochemical analysis unit. As in the cases of an enzyme-labeled antibody, which will be described later, the blocking agent should preferably be subjected to filtration before being used.

After the labeled receptor or the labeled ligand has been specifically bound to at least one of the ligands, each of which has been bound to one of the porous adsorptive regions of the biochemical analysis unit, or at least one of the receptors, each of which has been bound to one of the porous adsorptive regions of the biochemical analysis unit, the biochemical analysis unit is set within, for example, the reaction vessel, which is illustrated in Figure 2 or Figure 3 and in which the reaction liquid is capable of being forcibly caused to flow such that the reaction liquid flows across each of the adsorptive regions of the biochemical analysis unit. In cases where the labeled receptor or the labeled ligand is a substance, such as a labeled DNA, which does not clog the absorptive regions, the setting of the biochemical analysis unit within the reaction vessel may be performed at the stage of subjecting the labeled receptor or the labeled ligand to the specific binding with the ligands or the receptors, which have been bound respectively to the porous adsorptive regions. In such cases, the labeled receptor or the labeled ligand is capable of being efficiently and uniformly brought into contact with the ligands or the receptors, which have been bound respectively to the porous adsorptive regions. Therefore, the limit of detection of the labeled receptor or the labeled ligand is capable of being enhanced. Also, in cases where the amount of the labeled receptor or the labeled ligand is small, the labeled receptor or the labeled ligand is capable of being detected with a high signal-to-noise ratio.

Further, the operations ranging from the stage of subjecting the labeled receptor or the labeled ligand to the specific binding with the ligands or the receptors, which have been bound respectively to the porous adsorptive regions, to the stage of subjecting the enzyme-labeled antibody to the specific binding with the labeled receptor or the labeled ligand, which has been specifically bound to at least one of the ligands or at least one of the receptors, are capable of being performed with the techniques for forcibly causing the reaction liquid, which contains the labeled receptor or the labeled ligand, to flow across each of the adsorptive regions and forcibly causing the reaction liquid, which contains the enzyme-labeled antibody, to flow across each of the adsorptive regions. Therefore, processes are capable of being simplified, and accurate detection is capable of being performed.

As described above, the stage of subjecting the labeled receptor or the labeled ligand to the specific binding with the ligands or the receptors, which have been bound respectively to the porous adsorptive regions, may be performed with the technique for forcibly causing the reaction liquid, which contains the labeled receptor or the labeled ligand, to flow across each of the adsorptive regions by use of, for example, the reaction vessel illustrated in Figure 2 or Figure 3. In such cases, the stage of bringing the blocking agent into contact with the adsorptive regions of the biochemical analysis unit, which stage is to be performed prior to the stage of subjecting the labeled receptor or the labeled ligand to the specific binding with the ligands or the receptors having been bound respectively to the porous adsorptive regions, is also capable of being performed with the technique for forcibly causing the reaction liquid, which contains the blocking agent, to flow by use of the reaction vessel. In such cases, the processes are capable of being simplified. Also, accurate data is capable of being obtained with good reproducibility and good quantitative characteristics.

In order for the labeled receptor or the labeled ligand, which has not been specifically bound to the ligands or the receptors havingbeenbound respectively to the porous adsorptive regions of the biochemical analysis unit, to be removed, the biochemical analysis unit having been set within the reaction vessel should preferably be washed with a technique for forcibly causing a washing liquid to flow across each of the adsorptive regions. In such cases, since the washing liquid is forcibly caused to flow across each of the adsorptive regions, the labeled receptor or the labeled ligand, which has not been specifically bound to the ligands or the receptors having been bound respectively to the porous adsorptive regions of the biochemical analysis unit, is capable of being peeled off and removed efficiently. Therefore, the washing efficiency is capable of being enhanced markedly.

After the reaction liquid, which contains the enzyme-labeled antibody, is forcibly caused to flow such that the reaction liquid flows across each of the adsorptive regions of the biochemical analysis unit, andtheenzyme-labeledantibody is thus subjected to the specific binding with the labeled receptor or the labeled ligand, the enzyme-labeled antibody, which has not been specifically bound to the labeled receptor or the labeled ligand, may be removed. In cases where the enzyme-labeled antibody, which has not been specifically bound to the labeled receptor or the labeled ligand, is to be removed, the washing process described above should preferably be performed. In this manner, the enzyme-labeled antibody, which has not been specifically bound to the labeled receptor or the labeled ligand, is capable of being peeled off and removed efficiently. Therefore, the washing efficiency is capable of being enhanced markedly.

Before the enzyme-labeled antibody is subjected to the specific binding with the labeled receptor or the labeled ligand having been specifically bound to at least one of the ligands, each of which has been bound to one of the porous adsorptive regions of the biochemical analysis unit, or at least one of the receptors, each of which has been bound to one of the porous adsorptive regions of the biochemical analysis unit, the enzyme-labeled antibody should preferably be subjected to filtration using a filter having a pore diameter smaller than the pore diameter of the adsorptive regions. Since the enzyme-labeled antibody is apt to agglomerate, there is the risk that the enzyme-labeled antibody will clog the porous adsorptive regions. However, in cases where the enzyme-labeled antibody is subjected to the filtration in the manner described above, the enzyme-labeled antibody is capable of sufficiently penetrating into the interior of each of the adsorptive regions.

The pore diameter of the adsorptive regions is capable of being measured with a porous material automatic pore measuring system supplied by Porous Materials Inc. The pore diameter of the adsorptive regions may be measured in the manner described below. Specifically, a porous adsorptive region is wetted with a test liquid. Also, air is fed to the wetted porous adsorptive region, and the air pressure is raised little by little. In this manner, an air permeation flow rate is measured. (A wet flow rate curve is thus formed.) Further, in the state in which the adsorptive region is not wetted with the test liquid, the air permeation flow rate is measured at the same pressure. (A dry flow rate curve is thus formed.) The wet flow rate and the dry flow rate are then compared with each other. (Specifically, the pressure associated with a point, at which a curve having an inclination of one half of the inclination of the dry flow rate curve and the wet flow rate curve intersect with each other, is found.) A mean pore diameter is then capable of being calculated from a relation between the pressure and the pore diameter. Ordinarily, the pore diameter of the filter for filtering the enzyme-labeled antibody is defined in accordance with whether particles having a certain size are or are not capable of passing through the filter. Therefore, a filter, which has a pore diameter smaller than the pore diameter of the adsorptive regions having been measured in the manner described above, may be selected and utilized for filtering the enzyme-labeled antibody. The pore diameter of the selected filter should preferably be at most one half of the pore diameter of the adsorptive regions.

Thereafter, the reaction liquid, which contains the enzyme-labeled antibody, is forcibly caused to flow such that the reaction liquid flows across each of the adsorptive regions of the biochemical analysis unit, and the enzyme-labeled antibody is thus subjected to the specific binding with the labeled receptor or the labeled ligand. After the reaction liquid containing the enzyme-labeled antibody has been forcibly caused to flow such that the reaction liquid flows across each of the porous adsorptive regions of the biochemical analysis unit, the forcible flowing should preferably be ceased during a period of time longer than the period of time during which the reaction liquid containing the enzyme-labeled antibody has been forcibly caused to flow. The period of time, during which the reaction liquid containing the enzyme-labeled antibody is forcibly caused to flow, and the period of time, during which the forcible flowing is ceased, may vary in accordance with the kind of the ligand or the receptor, the kind of the labeled receptor or the labeled ligand, the quantity of the ligand or the receptor, the quantity of the labeled receptor or the labeled ligand, and the like. However, in cases where the period of time, during which the reaction liquid containing the enzyme-labeled antibody is forcibly caused to flow, falls within the range of one minute to 30 minutes, the period of time, during which the forcible flowing is ceased, should preferably fall within the range of 30 minutes to one hour. Alternatively, a cycle comprising the forcible flowing of the reaction liquid, the enzyme-labeled antibody, and the ceasing of the flowing of the reaction liquid may be iterated. Specifically, for example, the forcible flowing of the reaction liquid, which contains the enzyme-labeled antibody, may be performed for one minute, the flowing of the reaction liquid may be ceased for 30 minutes, the forcible flowing of the reaction liquid may then be performed for one minute, and the flowing of the reaction liquid may thereafter be ceased for 30 minutes.

The enzyme-labeled antibody is the antibody with respect to the labeling substance of the labeled receptor or the labeled ligand, which antibody has been labeled with an enzyme. (In cases where the labeling substance of the labeled receptor or the labeled ligand is an antibody, the enzyme-labeled antibody is the antigen with respect to the labeling substance of the labeled receptor or the labeled ligand, which antigen has been labeled with an enzyme.) Examples of the enzymes preferable as the enzyme of the enzyme-labeled antibody include alkaline phosphatase, peroxidase, and luciferase.

Thereafter, the biochemical analysis unit is taken out from the reaction vessel, and a chemical luminescence substrate is brought into contact with the enzyme-labeled antibody, which has been specifically bound to the labeled receptor or the labeled ligand. In cases where the enzyme of the enzyme-labeled antibody is alkaline phosphatase, the chemical luminescence substrate caused to undergo the reaction with the enzyme-labeled antibody may be dioxetane. In cases where the enzyme of the enzyme-labeled antibody is peroxidase, the chemical luminescence substrate caused to undergo the reaction with the enzyme-labeled antibody may be luminol. Also, in where the enzyme of the enzyme-labeled antibody is luciferase, the chemical luminescence substrate caused to undergo the reaction with the enzyme-labeled antibody may be luciferin. However, the chemical luminescence substrate caused to undergo the reaction with the enzyme-labeled antibody is not limited to the chemical luminescence substrates enumerated above.

In cases where the chemical luminescence substrate and the enzyme are brought into contact with each other, the chemical luminescence having wavelengths falling within the visible light wavelength range is produced. Therefore, the produced chemical luminescence may be detected photoelectrically, and the image data for a biochemical analysis may be formed in accordance with the detected chemical luminescence. In this manner, the labeled receptor or the labeled ligand is capable of being detected and determined.

The present invention will further be illustrated by the following nonlimitative examples.

### Examples

### Example 1

With an etching technique, 400 fine holes were formed in a SUS304 sheet (acting as a base plate material sheet having a size of 90mm x 90mm and a thickness of 100µm. Each of the fine holes had a circular opening region having a hole diameter of 0.3mm. The fine holes were formed at a hole pitch of 0.4mm and a hole spacing of 0.1mm.

Thereafter, an adhesive agent was applied to one surface of the base plate material sheet, and the adhesive agent, which entered into the holes having been formed in the base plate material sheet, was removed by suction. The adhesive agent remaining on the surface of the base plate material sheet was then dried. Thereafter, a 6, 6-nylon membrane having a pore size of 0.45µm and a thickness of 170µm was superposed upon the surface of the base plate material sheet, which surface had been coated with the adhesive agent. The combination of the 6,6-nylon membrane and the base plate material sheet was then heated to a temperature of 150°C and pressed under pressure such that the pressure per 1cm² was 300kg. The 6,6-nylon membrane was thus press-fitted into the fine holes of the base plate material sheet. In this manner, a biochemical analysis unit, which comprised a stainless steel barrier wall and the plurality of polymer-filled regions formed in the fine holes, was prepared.

Also, a digoxigenin-labeled pBR328-DNA liquid (supplied by Roche Diagnostics K.K.) was diluted with a TE buffer (a mixed solution of 10mM of Tris-HCL and 1mM of EDTA, supplied by Nippon Gene K.K.) to a concentration of 50pg/µl. Thermal denaturation was then performed, and the digoxiganin-labeled pBR328-DNA was thus converted into a single stranded form. Thereafter, 10nl of the digoxigenin-labeled pBR328-DNA liquid was spotted onto each of the adsorptive regions of the biochemical analysis unit having been prepared in the same manner as that described above. Thereafter, with irradiation of ultraviolet light (254nm, 33mJ/cm²), the single stranded digoxigenin-labeled pBR328/BgII,HinfI was fixed to the adsorptive regions of the biochemical analysis unit.

Thereafter, the biochemical analysis unit was set in the reaction vessel illustrated in Figure 2. Also, a washing buffer (supplied by Roche Diagnostics K.K.) was diluted with sterilized deionized water to a concentration of 1/10, and a washing liquid was thereby prepared. The thus prepared washing liquid was fed into the reaction vessel, in which the biochemical analysis unit had been accommodated. The pump was actuated, and the adsorptive regions of the biochemical analysis unit were washed with the washing liquid.

Thereafter, by use of a maleic acid buffer (supplied by Roche Diagnostics K.K.), which had been diluted with sterilized deionized water to a concentration of 1/10, a blocking buffer solution ((supplied by Roche Diagnostics K. K.) was diluted to a concentration of 1/10. The thus diluted blocking buffer solution was then subjected to filtration with a polyether sulfone filter (pore diameter: 0.2µm) and then utilized as a blocking agent. After the washing liquid had been discharged from the reaction vessel, the blocking agent was fed into the reaction vessel, and the pump was driven for a predetermined periodoftime. Thereafter, the operation of the pump was ceased, and the blocking agent was allowed to stand for a predetermined period of time within the reaction vessel. In this manner, a blocking reaction was performed.

Thereafter, an anti-digoxigenin-AP-conjugate (an alkaline phosphatase-labeled digoxigenin antibody) was subjected to centrifugal filtration with a polyvinylidene fluoride filter (pore diameter: 0.2µm). The anti-digoxigenin-AP-conjugate having been collected by filtration was then diluted with the aforesaid blocking agent to a concentration of 1/10, 000, and an enzyme-labeled antibody liquid was thereby prepared. After the blocking agent had been discharged from the reaction vessel, the thus prepared enzyme-labeled antibody liquid was fed into the reaction vessel, and the pump was driven for a predetermined period of time. Thereafter, the operation of the pump was ceased, and the enzyme-labeled antibody liquid was allowed to stand for a predetermined period of time within the reaction vessel. In this manner, an antigen-antibody reaction was performed.

After the antigen-antibody reaction was completed, the washing buffer was fed into the reaction vessel. Also, the pump was driven, and the adsorptive regions of the biochemical analysis unit were washed for 15 minutes. The washing operation was iterated three times. The biochemical analysis unit was taken out from the reaction vessel and was then brought into contact with a liquid containing a chemical luminescence substrate (CDP-star, ready to use, supplied by Roche Diagnostics K.K.). Also, the chemical luminescence, which was emitted from the adsorptive regions of the biochemical analysis unit, was detected photoelectrically by use of a cooled CCD camera (LAS1000, supplied by Fuji Photo FilmCo. , Ltd.). In this manner, a digital signal was formed.

### Comparative Example 1

A biochemical analysis unit having the adsorptive regions, to which the single stranded digoxigenin-labeled pBR328/BgII,HinfI was fixed as in Example 1, was put in a hybridization bag. The same washing liquid as that in Example 1 was fed into the hybridization bag. Vibrations were given to the hybridization bag, and shaking of the hybridization bag was thus performed for five minutes. Thereafter, the washing liquid was discharged from the hybridization bag, and the same blocking agent as that in Example 1 was fed into the hybridization bag. Shaking of the hybridization bag was then performed for one hour, and a blocking reaction was thus performed. Thereafter, the same enzyme-labeled antibody liquid as that in Example 1 was fed into the hybridization bag, and shaking of the hybridization bag was performed for one hour. In this manner, an antigen-antibody reactius, was performed, After the antigen-antibody reaction was completed, the washing buffer was fed into the hybridization bag, and washing was performed with shaking for 15 minutes. The washing operation was iterated three times. After the washing operation was finished, the biochemical analysis unit was taken out from the hybridization bag and was then brought into contact with a liquid containing a chemical luminescence substrate (CDP-star, ready to use, supplied by Roche Diagnostics K.K.). Also, the chemical luminescence, which was emitted from the adsorptive regions of the biochemical analysis unit, was detected photoelectrically by use of a cooled CCD camera (LAS1000, supplied by Fuji Photo Film Co., Ltd.). In this manner, a digital signal was formed.

In Example 1, the pump driving time and the standing time for the blocking reaction and the pump driving time and the standing time for the antigen-antibody reaction were set at various periods of time. In such cases, intensities of the background, intensities of the digital signal, and the signal-to-noise ratios (S/N ratios) listed in Table 1 below were obtained. Also, in Comparative Example 1, in which the conventional technique for shaking the hybridization bag was employed, the intensity of the background, the intensity of the digital signal, and the signal-to-noise ratio (S/N ratio) listed in Table 1 below were obtained.

**Table 1**

| | Blocking reaction | | Antigen-antibody | | Detection results | | |
|---|---|---|---|---|---|---|---|
| | | | reaction | | | | |
| | Pump driving time | Standing time | Pump driving time | Standing time | Back-ground | Signal | S/N ratio |
| Ex. 1 | 10 min | 50 min | 1 min | 1 hr | 1900 | 21800 | 11.4 |
| | 30 min | 30 min | 1 min | 1 hr | 3000 | 23000 | 7.7 |
| | 60 min | 0 min | 1 min | 1 hr | 4400 | 19000 | 4.3 |
| | 10 min | 50 min | 5 min | 55 min | 2300 | 15500 | 6.7 |
| | 10 min | 50 min | 15 min | 45 min | 1900 | 11400 | 6 |
| | 10 min | 50 min | 30 min | 30 min | 2100 | 8800 | 4.2 |
| Comp. Ex. 1 | - | - | - | - | 6100 | 13700 | 2.2 |

As described above, in Example 1, at the time at which the enzyme-labeled antibody is subjected to the specific binding with the labeled receptor or the labeled ligand, which has been specifically bound to at least one of the ligands or at least one of the receptors, the reaction liquid containing the enzyme-labeled antibody is forcibly caused to flow such that the reaction liquid containing the enzyme-labeled antibody flows across each of the porous adsorptive regions of the biochemical analysis unit. Therefore, as clear from Table 1, in Example 1, wherein the reaction liquid containing the enzyme-labeled antibody is forcibly caused to flow such that the reaction liquid containing the enzyme-labeled antibody flows across each of the porous adsorptive regions of the biochemical analysis unit, the enzyme-labeled antibody is capable of being caused to penetrate sufficiently into the interior of each of the adsorptive regions . Accordingly, regardless of the length of time during which the reaction liquid is forcibly caused to flow, the signal having a higher intensity is capable of being detected than in Comparative Example 1, in which the conventional technique of shaking the hybridization bag, and the intensity of the background was lower than in Comparative Example 1.

Also, as clear from Table 1, the intensity of the signal obtained from the antigen-antibody reaction is affected more largely by the length of the standing time for the antigen-antibody reaction after the driving of the pump than by the length of the pump driving time. Thisispresumablybecause, in cases where, after the reaction liquid containing the enzyme-labeled antibody has been forcibly caused to flow such that the reaction liquid flows across each of the porous adsorptive regions of the biochemical analysis unit, the forcible flowing is ceased in this state, more specifically in cases where the forcible flowing is ceased during the period of time longer than the period of time during which the reaction liquid containing the enzyme-labeled antibody has been forcibly caused to flow, the enzyme-labeled antibody is capable of being sufficiently in contact with the labeled receptor or the labeled ligand, the binding of the enzyme-labeled antibody with the labeled receptor or the labeled ligand is capable of being performed firmly, and the detection is capable of being performed with a high signal-to-noise ratio.

Also, as for the blocking reaction, in cases where, after the reaction liquid containing the blocking agent has been forcibly caused to flow such that the reaction liquid flows across each of the porous adsorptive regions of the biochemical analysis unit, the forcible flowing is ceased in this state, the intensity of the background detected is low. This is presumably because, in cases where the reaction liquid containing the blocking agent is thus allowed to stand, the blocking agent is capable of being bound or adsorbed more reliably to the adsorptive regions.

### Example 2

After a molecular weight marker pBR328/BgII,HinfI (25ng/µl, supplied by Roche Diagnostics K.K.) having been dissolved in the TE buffer was boiled for five minutes, the liquid was cooled for one minute in an ice-bath, and the pBR328/BgII, HinfI was thus converted into a single stranded form. The thus obtained pBR328/BgII, HinfI liquid was then spotted onto the adsorptive regions of the biochemical analysis unit having been prepared in the samemanneras that in Example 1. Thereafter, with irradiation of ultraviolet light (254nm, 33mJ/cm²), the single stranded pBR328/BgII,HinfI was fixed to the adsorptive regions of the biochemical analysis unit.

Thereafter, a hybridization liquid was prepared. The hybridization liquid had the composition such that 100ml of the hybridization liquid contained 52ml of sterilized deionized water, 30ml of 20xSSC (supplied by Nippon Gene K.K.), 2ml of 0.5M EDTA (pH 8.0), 10ml of a 50xdenhard' s liquid, 5ml of a 10% SDS liquid, and 1ml of a salmon spermatozoon denatured DNA having a concentration of 100µg/ml.

Also, a digoxigenin-labeled pBR328-DNA liquid having a concentration of 5ng/µl was diluted with the TE buffer, and the concentration of the digoxigenin-labeled pBR328-DNA liquid was adjusted to a predetermined value. Thermal denaturation was then performed, and the digoxigenin-labeled pBR328-DNA was thus converted into a single stranded form. Thereafter, the digoxigenin-labeled pBR328-DNA liquid was diluted even further with the hybridization liquid. In this manner, a digoxigenin-labeled pBR328-DNA liquid having a predetermined concentration (i.e., a hybridization reaction liquid) was prepared.

Thereafter, the biochemical analysis unit described above was put in a hybridization bag, and the hybridization reaction liquid having been prepared in the manner described above was filled in the hybridization bag. Vibrations were then given to the hybridization bag, and shaking was thus performed for 18 hours. In this manner, a hybridization reaction was performed.

The biochemical analysis unit described above was then fixed in the reaction vessel illustrated in Figure 2. Thereafter, the washing operation, the blocking reaction, and the antigen-antibody reaction were performed in the same manner as that in Example 1. The biochemical analysis unit was thenbrought into contact with the chemical luminescence substrate in the samemanner as that in Example 1. Also, the chemical luminescence, which was emitted from the adsorptive regions of the biochemical analysis unit, was detected photoelectrically by use of the cooled CCD camera (LAS1000). In this manner, a digital signal was formed.

### Example 3

The same procedure as that in Example 2 was performed, except that the hybridization reaction was performed in the reaction vessel illustrated in Figure 2 for 18 hours by driving the pump. The washing operation, the blocking reaction, the antigen-antibody reaction, and the operation for bringing the biochemical analysis unit into contact with the chemical luminescence substrate were performed in the same manner as that in Example 2. Also, the chemical luminescence, which was emitted from the adsorptive regions of the biochemical analysis unit, was detected photoelectrically by use of the cooled CCD camera (LAS1000). In this manner, a digital signal was formed.

### Comparative Example 2

After the hybridization reaction was performed in the same manner as that in Example 2, the biochemical analysis unit was kept within the hybridization bag, the same washing liquid as that in Example 2 was fed into the hybridization bag, and the shaking was performed for five minutes. Thereafter, the washing liquid was discharged from the hybridization bag. Also, the same enzyme-labeled antibody liquid as that in Example 1 was fed into the hybridization bag, the shaking was performed for one hour, and the antigen-antibody reaction was thus performed. After the antigen-antibody reaction was completed, the washing buffer was fed into the hybridization bag, and washing was performed with shaking for 15 minutes. The washing operation was iterated three times. After the washing operation was finished, the biochemical analysis unit was taken out from the hybridization bag and was then brought into contact with a liquid containing the chemical luminescence substrate (CDP-star). Also, the chemical luminescence, which was emitted from the adsorptive regions of the biochemical analysis unit, was detected photoelectrically by use of the cooled CCD camera (LAS1000). In this manner, a digital signal was formed.

In each of Example 1, Example 2, Example 3, and Comparative Example 2, the amount of the digoxigenin-labeled pBR328-DNAwas set at various values. In such cases, intensities of the background, intensities of the digital signal, and the signal-to-noise ratios (S/N ratios) listed in Table 2 below were obtained.

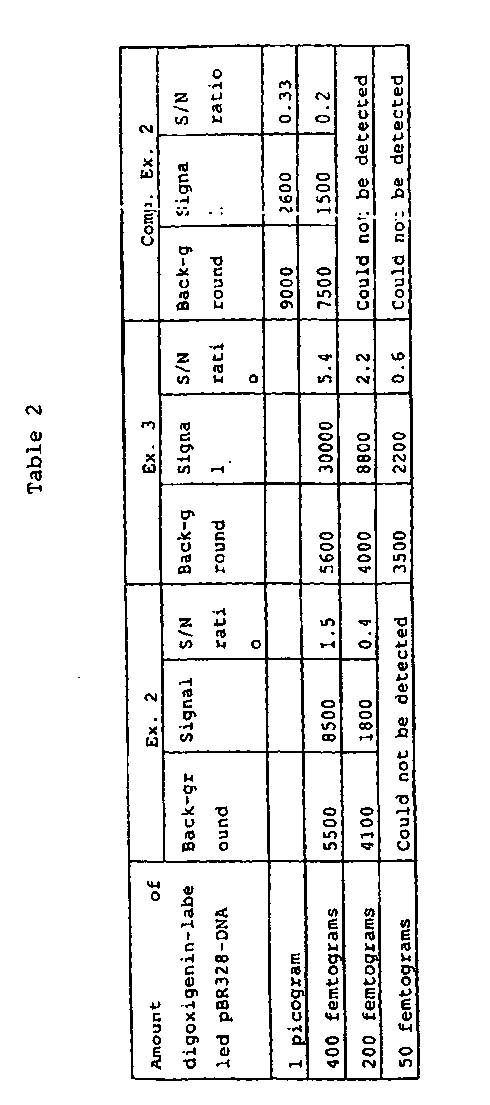

As described above, in Example 2, the hybridization reaction was performed with the conventional technique for shaking the hybridization bag, and the antigen-antibody reaction was performed with the reaction apparatus in accordance with the present invention. In Example 3, both the hybridization reaction and the antigen-antibody reaction were performed with the reaction apparatus in accordance with the present invention. Also, in Comparative Example 2, both the hybridization reaction and the antigen-antibody reaction were performed with the conventional technique for shaking the hybridization bag. As clear from Table 2, in Example 2 and Example 3, a markedly smaller amount of the digoxigenin-labeled pBR328-DNA is capable of being detected with a higher signal-to-noise ratio than in Comparative Example 2.

As described above, with the chemical luminescence method using a biochemical analysis unit in accordance with the present invention, at the time at which the enzyme-labeled antibody is subjected to the specific binding with the labeled receptor or the labeled ligand, which has been specifically bound to at least one of the ligands or at least one of the receptors, the reaction liquid containing the enzyme-labeled antibody is forcibly caused to flow such that the reaction liquid containing the enzyme-labeled antibody flows across each of the porous adsorptive regions of the biochemical analysis unit. Therefore, the enzyme-labeled antibody is capable of being caused to penetrate sufficiently into the interior of each of the adsorptive regions. Accordingly, in cases where the amount of the labeled receptor or the labeled ligand is small, the labeled receptor or the labeled ligand is capable of being detected with a high signal-to-noise ratio.

In Example 3, also at the time at which the labeled receptor or the labeled ligand having been labeled with the labeling substance was subjected to the specific binding with the ligands of the receptors, the reaction liquid containing the labeled receptor or the labeled ligand, which had been labeled with the labeling substance, was forcibly caused to flow such that the reaction liquid containing the labeled receptor or the labeled ligand flowed across each of the porous adsorptive regions of the biochemical analysis unit. Therefore, as clear from Table 2, in cases where the amount of the labeled receptor or the labeled ligand is markedly small, the labeled receptor or the labeled ligand is capable of being detected with a high signal-to-noise ratio.

Further, in cases where, after the reaction liquid containing the enzyme-labeled antibody has been forcibly caused to flow such that the reaction liquid flows across each of the porous adsorptive regions of the biochemical analysis unit, the forcible flowing is ceased during the period of time longer than the period of time during which the reaction liquid containing the enzyme-labeled antibody has been forcibly caused to flow, the labeled receptor or the labeled ligand is capable of being detected with a high signal-to-noise ratio.

## Claims

1. A chemical luminescence method using a biochemical analysis unit, comprising the steps of:
i) obtaining a biochemical analysis unit provided with a plurality of porous adsorptive regions, to which ligands or receptors have been bound respectively,
ii) subjecting a labeled receptor or a labeled ligand, which has been labeled with a labeling substance, to specific binding with the ligands or the receptors, each of which has been bound to one of the porous adsorptive regions of the biochemical analysis unit, the labeled receptor or the labeled ligand being thereby specifically bound to at least one of the ligands or at least one of the receptors,
iii) subjecting an enzyme-labeled antibody to specificbindingwith the labeled receptor or the labeled ligand, which has been specifically bound to at least one of the ligands or at least one of the receptors, and
iv) causing a chemical luminescence substrate to undergo a reaction with the enzyme-labeled antibody, which has been specifically bound to the labeled receptor or the labeled ligand,
wherein, at the time at which the enzyme-labeled antibody is subjected to the specific binding with the labeled receptor or the labeled ligand, which has been specifically bound to at least one of the ligands or at least one of the receptors, a reaction liquid containing the enzyme-labeled antibody is forced to flow in a manner that causes the reaction liquid containing the enzyme-labeled antibody to flow across each of the porous adsorptive regions of the biochemical analysis unit, and the forced flowing is ceased during a period of time longer than the period of time during which the reaction liquid containing the enzyme-labeled antibody has been forced to flow before srep iv.

2. A method according to claim 1 wherein, at the time at which the labeled receptor or the labeled ligand having been labeled with the labeling substance is subjected to the specific binding with the ligands or the receptors, each of which has been bound to one of the porous adsorptive regions of the biochemical analysis unit, a reaction liquid containing the labeled receptor or the labeled ligand, which has been labeled with the labeling substance, is forced to flow in a manner that causes the reaction liquid containing the labeled receptor or the labeled ligand to flow across each of the porous adsorptive regions of the biochemical analysis unit.

## Revendications

1. Procédé de chimioluminescence utilisant un module d'analyse biochimique, comprenant les étapes consistant à :
i) obtenir un module d'analyse biochimique muni d'une pluralité de régions adsorbantes poreuses auxquelles des ligands ou des récepteurs ont été respectivement liés,
ii) soumettre un récepteur marqué ou un ligand marqué, qui a été marqué avec une substance de marquage, à une liaison spécifique avec les ligands ou les récepteurs, dont chacun s'est lié à l'une des régions adsorbantes poreuses du module d'analyse biochimique, le récepteur marqué ou le ligand marqué étant ainsi liés spécifiquement à l'un au moins des ligands ou à l'un au moins des récepteurs,
iii) soumettre un anticorps marqué par une enzyme à une liaison spécifique avec le récepteur marqué ou le ligand marqué, qui s'est lié spécifiquement à l'un au moins des ligands ou à l'un au moins des récepteurs, et
iv) faire réagir un substrat chimioluminescent avec l'anticorps marqué par une enzyme, qui s'est lié spécifiquement au récepteur marqué ou au ligand marqué,
dans lequel, au moment où l'anticorps marqué par une enzyme est soumis à la liaison spécifique avec le récepteur marqué ou le ligand marqué, qui s'est lié spécifiquement à l'un au moins des ligands ou à l'un au moins des récepteurs, un liquide réactionnel contenant l'anticorps marqué par une enzyme est forcé à s'écouler d'une manière qui amène le liquide réactionnel contenant l'anticorps marqué par une enzyme à s'écouler dans chacune des régions adsorbantes poreuses du module d'analyse biochimique et l'écoulement forcé est stoppé pendant une durée supérieure à la durée pendant laquelle le liquide réactionnel contenant l'anticorps marqué par une enzyme a été forcé à s'écouler avant l'étape iv).

2. Procédé selon la revendication 1, dans lequel, au moment où le récepteur marqué ou le ligand marqué qui a été marqué avec la substance de marquage est soumis à la liaison spécifique avec les ligands ou les récepteurs, dont chacun s'est lié à l'une des régions adsorbantes poreuses du module d'analyse biochimique, un liquide réactionnel contenant le récepteur marqué ou le ligand marqué, qui a été marqué avec la substance de marquage, est forcé à s'écouler d'une manière qui amène le liquide réactionnel contenant le récepteur marqué ou le ligand marqué à s'écouler dans chacune des régions adsorbantes poreuses du module d'analyse biochimique.

## Patentansprüche

1. Chemisches Lumineszenzverfahren, das eine biochemische Analyseeinheit verwendet, folgende Schritte aufweisend:
i) Erhalten einer biochemischen Analyseeinheit, die mit einer Mehrzahl poröser adsorptionsfähiger Bereiche ausgestattet ist, an die Liganden bzw. Rezeptoren gebunden wurden,
ii) Unterziehen eines markierten Rezeptors oder eines markierten Liganden, der mit einer Markierungssubstanz markiert wurde, einer spezifischen Bindung mit den Liganden oder den Rezeptoren, von denen jeder an einen der porösen adsorptionsfähigen Bereiche der biochemischen Analyseeinheit gebunden wurde, wodurch der markierte Rezeptor oder der markierte Ligand an mindestens einen der Liganden oder mindestens einen der Rezeptoren spezifisch gebunden wird,
iii) Unterziehen eines Enzym-markierten Antikörpers einer spezifischen Bindung mit dem markierten Rezeptor oder dem markierten Liganden,der an mindestens einen der Liganden oder mindestens einen der Rezeptoren spezifisch gebunden wurde, und
iv) Veranlassen' eines chemischen Lumineszenz-Substrats, eine Reaktion mit dem Enzym-markierten Antikörper, der an den markierten Rezeptor oder den markierten Liganden spezifisch gebunden wurde, einzugehen,
wobei zu der Zeit, zu der der Enzym-markierte Antikörper der spezifischen Bindung mit dem markierten Rezeptor oder dem markierten Liganden, der an mindestens einen der Liganden oder mindestens einen der Rezeptoren spezifisch gebunden wurde, unterzogen wird, eine den Enzym-markierten Antikörper enthaltende Reaktionsflüssigkeit in einer Weise zum Fließen gezwungen wird, die die Reaktionsflüssigkeit, die den Enzym-markierten Antikörper enthält, dazu ver- . anlaßt, durch jeden der porösen adsorptionsfähigen Bereiche der biochemischen Analyseeinheit zu fließen, und wobei das erzwungene Fließen während einer Zeitdauer, die länger ist als die Zeitdauer, während der die den Enzym-markierten Antikörper enthaltende Reaktionsflüssigkeit zum Fließen gezwungen wurde, eingestellt wird, vor Schritt iv.

2. Verfahren nach Anspruch 1, bei dem zu der Zeit, zu der der markierte Rezeptor oder der markierte Ligand, der mit der Markierungssubstanz markiert wurde, der spezifischen Bindung mit den Liganden oder den Rezeptoren, von denen jeder an einen der porösen adsorptionsfähigen Bereiche der biochemischen Analyseeinheit gebunden wurde, unterzogen wird, eine Reaktionsflüssigkeit, die den markierten Rezeptor oder den markierten Liganden, der mit der Markierungssubstanz markiert wurde, enthält, in einer Weise zum Fließen gezwungen wird, die die Reaktionsflüssigkeit, die den markierten Rezeptor oder den markierten Liganden enthält, dazu veranlaßt, durch jeden der porösen adsorptionsfähigen Bereiche der biochemischen Analyseeinheit zu fließen.
